# EUROPEAN PATENT SPECIFICATION

(11) **EP 3 990 419 B1**
(45) Date of publication and mention of the grant of the patent: **11.03.2026**
(21) Application number: 20734072.0
(22) Date of filing: 26.06.2020
(51) Int. Cl.: C07C 1/24, C07C 5/03, C07C 9/14, C07C 11/02, C07C 29/54, C07C 31/125, C07C 29/40

(54) **PROCESS FOR PRODUCING TRIPTANE AND/OR TRIPTENE**
VERFAHREN ZUR HERSTELLUNG VON TRIPTAN UND/ODER TRIPTEN
PROCÉDÉ DE PRODUCTION DE TRIPTANE ET/OU DE TRIPTÈNE

(30) Priority: 28.06.2019 EP 19183389
(43) Date of publication of application: 04.05.2022
(73) Proprietor: Shell Internationale Research Maatschappij B.V., 2596 HR The Hague (NL)
(72) Inventor: REDMANN, Jan-Hendrik, 22335 Hamburg (DE)
(74) Representative: Shell Legal Services IP
(86) International application number: PCT/EP2020/068060
(87) International publication number: WO 2020/260607

(56) References cited:
- GRAHAM EDGAR ET AL: "THE PREPARATION AND PROPERTIES OF THE ISOMERIC HEPTANES. PART I. PREPARATION1", J. AM. CHEM. SOC., vol. 51, no. 5, 1 January 1929 (1929-01-01), pages 1483 - 1491, XP055648421
- JOHN?H. AHN ET AL: "Selective Homologation Routes to 2,2,3-Trimethylbutane on Solid Acids", ANGEWANDTE CHEMIE, INTERNATIONAL EDITION, vol. 48, no. 21, 11 May 2009 (2009-05-11), DE, pages 3814 - 3816, XP055648428, ISSN: 1433-7851, DOI: 10.1002/anie.200900541

## Description

### Field of the Invention

The present invention relates to a process for the production of 2,2,3-trimethylbutane (also known as "triptane"). The present process can also be used to produce the C7 hydrocarbon 2,2,3-trimethylbut-1-ene (also known as "triptene"). In general, processes that produce triptane may also produce triptene, which may wholly or partly be converted to triptane during the process. Both have high octane numbers.

### Background of the Invention

It is known that certain branched hydrocarbons, and triptane in particular, provide high octane properties to fuels such as gasoline and aviation fuels. Triptane has been found to provide superior engine performance due to its high energy density and high octane number.

Various processes are known for the production of triptane. For example, WO2009/120508 discloses a process for the production of triptane and triptene by homologation of dimethyl ether and/or methanol.

G.Edgard, G.Calingaert and R.Marker "The Preparation and Properties of the Isomeric Heptanes Part I. Preparation" 1929 discloses that the six branched isomers of heptane can be prepared by creating a suitable secondary or tertiary alcohol by the Grignard reaction, converting it to an alkene by dehydration, and hydrogenating the latter. The paper discloses that the 2,2-dimethylpentane isomer can be prepared by reacting tert-butyl chloride with n-propyl magnesium bromide and the 3,3-dimethylpentane isomer can be prepared from tert-amyl chloride and ethyl magnesium bromide.

Since triptane is a useful, high octane gasoline blending component, it would be desirable to provide an improved process for synthesizing triptane, in particular involving higher yields and fewer side reactions.

### Summary of the Invention

According to the present invention there is provided a process for producing triptane and/or triptene, comprising the steps:
(i) reaction of tertiary butyl halide with magnesium to form a Grignard reagent;
(ii) reaction of the Grignard reagent from step (i) with acetone to form a Grignard adduct;
(iii) hydrolysation of the Grignard adduct to form a tertiary alcohol;
(iv) dehydration of the tertiary alcohol to form triptene; and preferably,
(v) hydrogenation of the C7 alkene produced in step (iv) to produce triptane;
characterised in that step (ii) takes place in a continuous reactor, wherein the continuous reactor is a milli-reactor, wherein step (i) takes place in a separate reactor to step (ii) and additionally wherein step (iii) takes place in a milli-reactor.

The present invention provides an improved process for producing triptane, in particular having an improved yield and fewer side reactions.

### Detailed Description of the Invention

The first step of the process of the present invention involves the reaction of a tertiary butyl halide with magnesium to form a Grignard reagent. The formation of Grignard reagents is a well known synthesis step known to those skilled in the art. In a preferred embodiment, the tertiary butyl halide is mixed with magnesium in a suitable solvent, such as anhydrous ethyl ether.

Any tertiary butyl halide which is capable of reacting with magnesium to form a Grignard reagent is suitable for use herein. Preferably, the tertiary butyl halide is selected from tertiary butyl chloride, tertiary butyl bromide and tertiary butyl iodide, and mixtures thereof. In a particularly preferred embodiment, the tertiary butyl halide is tertiary butyl chloride.

The second step of the process of the present invention involves the reaction of the Grignard reagent formed in step (i) with acetone to form a Grignard adduct. In the present process, this second step takes place in a continuous reactor preferably selected from a milli-reactor.

Milli- or micro- reactors are reactors in which continuous processes are able to take place in structures of greatly reduced size. While not wishing to be limited by theory it is believed that the use of a continuous reactor, preferably a milli-reactor or a micro-reactor, enables a short contact time and a controlled environment (no local hot spots) thereby preventing the formation of byproducts during the second step of the process. In addition, the use of a continuous reactor, preferably a milli-reactor or a micro-reactor, does not require a reduced temperature, which can be required for Grignard reactions carried out in batch reactors.

In a preferred embodiment, the second step of the process takes place in a milli-reactor. The milli-reactor will have sufficient length to allow for complete conversion.

In a preferred embodiment, the second step of the process is carried out at a temperature of from 5°C to 40°C.

In a preferred embodiment, the first step of the process is carried out in a separate reactor to the second step of the process in order to prevent pinacol coupling. In a preferred embodiment, step (i) will be carried out in a continuous column reactor in which magnesium is supplied from the top and tertiary butyl halide from the bottom. After formation of the Grignard reagent, the Grignard reagent will pass a filter to separate small magnesium particles and enter a continuous reactor, such as milli- reactor to react with acetone.

The third step of the process of the present invention involves the hydrolysation of the Grignard adduct to form a tertiary alcohol. The hydrolyzation reaction preferably takes place under acidic conditions. The organic phase from the second step of the process is preferably mixed with an acidic aqueous phase in order to hydrolyse the Grignard adduct and form the tertiary alcohol. In a preferred embodiment of the present invention, the third step of the process additionally takes place in a continuous reactor selected from a milli-reactor. In a particularly preferred embodiment of the present invention, the third step of the process takes place in a milli-reactor.

The fourth step of the process of the present invention involves dehydration of the tertiary alcohol formed in step (iii) to form triptene. The dehydration step typically happens immediately after step (iii).

The fifth step of the process of the present invention involves hydrogenation of the C7 alkene produced in step (iv) to produce triptane. The hydrogenation step is carried out in the presence of a hydrogenation catalyst such as nickel or palladium.

In a preferred embodiment, the organic phase is separated out at the end of step (iv) and is further separated by distillation. The solvent is preferably reused and the C7 alkene is preferably hydrogenated in a fully automated batch autoclave to form triptane.

The triptane and/or triptene produced by the process of the present invention can be used in a fuel composition. Hence according to another aspect of the present invention there is provided a fuel composition comprising the triptane and/or triptene produced by the process described herein.

Fuel compositions herein generally comprise a gasoline base fuel and optionally one or more fuel additives. Fuel compositions comprising a gasoline base fuel are therefore gasoline fuel compositions.

The gasoline fuel compositions herein comprise a gasoline base fuel. The gasoline base fuel may be any gasoline base fuel suitable for use in an internal combustion engine of the spark-ignition (gasoline) type known in the art, including automotive engines as well as in other types of engine such as, for example, off road and aviation engines. The gasoline used as the base fuel in the liquid fuel composition of the present invention may conveniently also be referred to as 'base gasoline'.

Gasolines typically comprise mixtures of hydrocarbons boiling in the range from 25 to 230°C (EN-ISO 3405), the optimal ranges and distillation curves typically varying according to climate and season of the year. The hydrocarbons in a gasoline may be derived by any means known in the art, conveniently the hydrocarbons may be derived in any known manner from straight-run gasoline, synthetically-produced aromatic hydrocarbon mixtures, thermally or catalytically cracked hydrocarbons, hydro-cracked petroleum fractions, catalytically reformed hydrocarbons or mixtures of these. All of these gasoline components may be derived from fossil carbon or renewables.

The specific distillation curve, hydrocarbon composition, research octane number (RON) and motor octane number (MON) of the gasoline are not critical for the present invention.

Conveniently, the research octane number (RON) of the gasoline may be at least 80, for instance in the range of from 80 to 110, preferably the RON of the gasoline will be at least 90, for instance in the range of from 90 to 110, more preferably the RON of the gasoline will be at least 91, for instance in the range of from 91 to 105, even more preferably the RON of the gasoline will be at least 92, for instance in the range of from 92 to 103, even more preferably the RON of the gasoline will be at least 93, for instance in the range of from 93 to 102, and most preferably the RON of the gasoline will be at least 94, for instance in the range of from 94 to 100 (EN 25164); the motor octane number (MON) of the gasoline may conveniently be at least 70, for instance in the range of from 70 to 110, preferably the MON of the gasoline will be at least 75, for instance in the range of from 75 to 105, more preferably the MON of the gasoline will be at least 80, for instance in the range of from 80 to 100, most preferably the MON of the gasoline will be at least 82, for instance in the range of from 82 to 95 (EN 25163).

Typically, gasolines comprise components selected from one or more of the following groups; saturated hydrocarbons, olefinic hydrocarbons, aromatic hydrocarbons, and oxygenated hydrocarbons. Conveniently, the gasoline may comprise a mixture of saturated hydrocarbons, olefinic hydrocarbons, aromatic hydrocarbons, and, optionally, oxygenated hydrocarbons.

Typically, the olefinic hydrocarbon content of the gasoline is in the range of from 0 to 40 percent by volume based on the gasoline (ASTM D1319); preferably, the olefinic hydrocarbon content of the gasoline is in the range of from 0 to 30 percent by volume based on the gasoline, more preferably, the olefinic hydrocarbon content of the gasoline is in the range of from 0 to 20 percent by volume based on the gasoline.

Typically, the aromatic hydrocarbon content of the gasoline is in the range of from 0 to 70 percent by volume based on the gasoline (ASTM D1319), for instance the aromatic hydrocarbon content of the gasoline is in the range of from 10 to 60 percent by volume based on the gasoline; preferably, the aromatic hydrocarbon content of the gasoline is in the range of from 0 to 50 percent by volume based on the gasoline, for instance the aromatic hydrocarbon content of the gasoline is in the range of from 10 to 50 percent by volume based on the gasoline.

The benzene content of the gasoline is at most 2 percent by volume, more preferably at most 1 percent by volume based on the gasoline.

The gasoline preferably has a low or ultra low sulphur content, for instance at most 1000 ppmw (parts per million by weight), preferably no more than 500 ppmw, more preferably no more than 100, even more preferably no more than 50 and most preferably no more than even 10 ppmw.

The gasoline also preferably has a low total lead content, such as at most 0.005 g/l, most preferably being lead free - having no lead compounds added thereto (i.e. unleaded).

When the gasoline comprises oxygenated hydrocarbons, at least a portion of non-oxygenated hydrocarbons will be substituted for oxygenated hydrocarbons. The oxygen content of the gasoline may be up to 35 percent by weight (EN 1601) (e.g. ethanol per se (i.e. pure anhydrous ethanol)) based on the gasoline. For example, the oxygen content of the gasoline may be up to 25 percent by weight, preferably up to 10 percent by weight. Conveniently, the oxygenate concentration will have a minimum concentration selected from any one of 0 and 5 percent by weight, and a maximum concentration selected from any one of 30, 20, 10 percent by weight. Preferably, the oxygenate concentration herein is 5 to 15 percent by weight.

Examples of oxygenated hydrocarbons that may be incorporated into the gasoline include alcohols, ethers, esters, ketones, aldehydes, carboxylic acids and their derivatives, and oxygen containing heterocyclic compounds. Preferably, the oxygenated hydrocarbons that may be incorporated into the gasoline are selected from alcohols (such as methanol, ethanol, propanol, 2-propanol, butanol, tert-butanol, iso-butanol and 2-butanol), ethers (preferably ethers containing 5 or more carbon atoms per molecule, e.g., methyl tert-butyl ether and ethyl tert-butyl ether) and esters (preferably esters containing 5 or more carbon atoms per molecule); a particularly preferred oxygenated hydrocarbon is ethanol.

When oxygenated hydrocarbons are present in the gasoline, the amount of oxygenated hydrocarbons in the gasoline may vary over a wide range. For example, gasolines comprising a major proportion of oxygenated hydrocarbons are currently commercially available in countries such as Brazil and U.S.A., e.g. ethanol per se and E85, as well as gasolines comprising a minor proportion of oxygenated hydrocarbons, e.g. E10 and E5. Therefore, the gasoline may contain up to 100 percent by volume oxygenated hydrocarbons. E100 fuels as used in Brazil are also included herein. Preferably, the amount of oxygenated hydrocarbons present in the gasoline is selected from one of the following amounts: up to 85 percent by volume; up to 70 percent by volume; up to 65 percent by volume; up to 30 percent by volume; up to 20 percent by volume; up to 15 percent by volume; and, up to 10 percent by volume, depending upon the desired final formulation of the gasoline. Conveniently, the gasoline may contain at least 0.5, 1.0 or 2.0 percent by volume oxygenated hydrocarbons.

Examples of suitable gasolines include gasolines which have an olefinic hydrocarbon content of from 0 to 20 percent by volume (ASTM D1319), an oxygen content of from 0 to 5 percent by weight (EN 1601), an aromatic hydrocarbon content of from 0 to 50 percent by volume (ASTM D1319) and a benzene content of at most 1 percent by volume.

Also suitable for use herein are gasoline blending components which can be derived from a biological source. Examples of such gasoline blending components can be found in WO2009/077606, WO2010/028206, WO2010/000761, European patent application nos. 09160983.4, 09176879.6, 09180904.6, and US patent application serial no. 61/312307.

Whilst not critical to the present invention, the base gasoline or the gasoline composition of the present invention may conveniently include one or more optional fuel additives. The concentration and nature of the optional fuel additive(s) that may be included in the base gasoline or the gasoline composition used in the present invention is not critical. Non-limiting examples of suitable types of fuel additives that can be included in the base gasoline or the gasoline composition used in the present invention include anti-oxidants, corrosion inhibitors, antiwear additives or surface modifiers, flame speed additives, detergents, dehazers, antiknock additives, metal deactivators, valve-seat recession protectant compounds, dyes, solvents, carrier fluids, diluents and markers. Examples of suitable such additives are described generally in
US Patent No. 5,855,629.

Conveniently, the fuel additives can be blended with one or more solvents to form an additive concentrate, the additive concentrate can then be admixed with the base gasoline or the gasoline composition of the present invention.

The (active matter) concentration of any optional additives present in the base gasoline or the gasoline composition of the present invention is preferably up to 1 percent by weight, more preferably in the range from 5 to 2000 ppmw, advantageously in the range of from 300 to 1500 ppmw, such as from 300 to 1000 ppmw.

The fuel compositions may be conveniently prepared using conventional formulation techniques by admixing one or more base fuels with the triptane and/or triptene produced according to the process of the present invention, together with one or more performance additive packages and/or one or more additive components.

## Claims

1. Process for producing triptane and/or triptene comprising the steps:
(i) reaction of tertiary butyl halide with magnesium to form a Grignard reagent;
(ii) reaction of the Grignard reagent from step (i) with acetone to form a Grignard adduct;
(iii) hydrolysation of the Grignard adduct to form a tertiary alcohol;
(iv) dehydration of the tertiary alcohol to form triptene; and preferably
(v) hydrogenation of the triptene produced in step (iv) to produce triptane;
**characterised in that** step (ii) takes place in a continuous reactor, wherein the continuous reactor is a milli-reactor, wherein step (i) takes place in a separate reactor to step (ii) and additionally wherein step (iii) takes place in a milli-reactor.

2. Process according to Claim 1 wherein step (ii) is carried out at a temperature of from 5°C to 40°C.

3. Process according to any of Claims 1 to 2 wherein the tertiary butyl halide is selected from tertiary butyl chloride, tertiary butyl bromide, tertiary butyl iodide, and mixtures thereof.

4. Process according to any of Claims 1 to 3 wherein the tertiary butyl halide is tertiary butyl chloride.

5. Process according to any of Claims 1 to 4 wherein step (v) takes place in the presence of a hydrogenation catalyst selected from nickel or palladium.

## Patentansprüche

1. Verfahren zum Herstellen von Triptan und/oder Tripten, umfassend die Schritte:
(i) Reagieren von tertiärem Butylhalogenid mit Magnesium, um ein Grignard-Reagenz auszubilden;
(ii) Reagieren des Grignard-Reagenzes aus Schritt
(i) mit Aceton, um ein Grignard-Addukt auszubilden;
(iii) Hydrolysieren des Grignard-Addukts, um einen tertiären Alkohol auszubilden;
(iv) Dehydratisieren des tertiären Alkohols, um Tripten auszubilden; und vorzugsweise
(v) Hydrieren des in Schritt (iv) erzeugten Triptens, um Triptan herzustellen;
**dadurch gekennzeichnet, dass** Schritt (ii) in einem kontinuierlichen Reaktor stattfindet, wobei der kontinuierliche Reaktor ein Milli-Reaktor ist, wobei Schritt (i) in einem separaten Reaktor von Schritt (ii) stattfindet und zusätzlich wobei Schritt (iii) in einem Milli-Reaktor stattfindet.

2. Verfahren nach Anspruch 1, wobei Schritt (ii) bei einer Temperatur von 5 °C bis 40 °C ausgeführt wird.

3. Verfahren nach einem der Ansprüche 1 bis 2, wobei das tertiäre Butylhalogenid ausgewählt ist aus tertiärem Butylchlorid, tertiärem Butylbromid, tertiärem Butyliodid und Mischungen davon.

4. Verfahren nach einem der Ansprüche 1 bis 3, wobei das tertiäre Butylhalogenid tertiäres Butylchlorid ist.

5. Verfahren nach einem der Ansprüche 1 bis 4, wobei Schritt (v) in Gegenwart eines Hydrierungskatalysators stattfindet, der aus Nickel oder Palladium ausgewählt ist.

## Revendications

1. Procédé de production de triptane et/ou de triptène comprenant les étapes de :
(i) réaction d'halogénure de butyle tertiaire avec du magnésium afin de former un réactif de Grignard ;
(ii)réaction du réactif de Grignard de l'étape (i) avec de l'acétone afin de former un adduit de Grignard ;
(iii) hydrolysation de l'adduit de Grignard afin de former un alcool tertiaire ;
(iv) déshydratation de l'alcool tertiaire afin de former du triptène ; et de préférence
(v) hydrogénation du triptène produit à l'étape (iv) afin de produire du triptane ;
**caractérisé en ce que** l'étape (ii) se déroule dans un réacteur continu, dans lequel le réacteur continu est un milli-réacteur, dans lequel l'étape (i) se déroule dans un réacteur séparé de l'étape (ii) et en outre dans lequel l'étape (iii) se déroule dans un milli-réacteur.

2. Procédé selon la revendication 1, dans lequel l'étape (ii) est effectuée à une température allant de 5 °C à 40 °C.

3. Procédé selon l'une quelconque des revendications 1 à 2, dans lequel l'halogénure de butyle tertiaire est choisi parmi chlorure de butyle tertiaire, bromure de butyle tertiaire, iodure de butyle tertiaire, et mélanges de ceux-ci.

4. Procédé selon l'une quelconque des revendications 1 à 3, dans lequel l'halogénure de butyle tertiaire est le chlorure de butyle tertiaire.

5. Procédé selon l'une quelconque des revendications 1 à 4, dans lequel l'étape (v) se déroule en présence d'un catalyseur d'hydrogénation choisi parmi le nickel ou le palladium.
